# EUROPÄISCHE PATENTSCHRIFT

(11) **EP 0 720 623 B1**
(45) Veröffentlichungstag und Bekanntmachung des Hinweises auf die Patenterteilung: **22.05.2002**
(21) Anmeldenummer: 94928798.1
(22) Anmeldetag: 22.09.1994
(51) Int. Cl.: C07K 16/28, A61K 39/395, G01N 33/577, C12P 21/08

(54) **MONOKLONALE ANTIKÖRPER GEGEN LEUKOZYTEN-SPEZIFISCHE G-PROTEIN-GEKOPPELTE REZEPTOREN**
MONOCLONAL ANTIBODIES AGAINST LEUKOCYTE-SPECIFIC G-PROTEIN-COUPLED RECEPTORS
ANTICORPS MONOCLONAUX DIRIGES CONTRE DES RECEPTEURS COUPLES A LA PROTEINE G SPECIFIQUES DES LEUCOCYTES

(30) Priorität: 22.09.1993 DE 4332256
(43) Veröffentlichungstag der Anmeldung: 10.07.1996
(73) Patentinhaber: Lipp, Martin, D-13507 Berlin - Tegel (DE)
(72) Erfinder: LIPP, Martin, D-13507 Berlin - Tegel (DE); FÖRSTER, Reinhold, D-81357 München (DE); EMRICH, Thomas, D-82393 Iffeldorf (DE); WOLF, Ingrid, D-81371 München (DE); KREMMER, Elisabeth, D-85354 Freising (DE)
(74) Vertreter: Baumbach, Friedrich
(86) Internationale Anmeldenummer: EP9403175
(87) Internationale Veröffentlichungsnummer: WO9508576

(56) Entgegenhaltungen:
- BIOLOGICAL CHEMISTRY HOPPE-SEYLER, Bd.373, Nr.9, September 1992, BRD Seite 840 I. WOLF ET AL. 'Differentiation-specific expression of a novel G protein-coupled receptor fromm Burkitt's lymphoma.'
- EUROPEAN JOURNAL OF IMMUNOLOGY, Bd.22, Nr.11, November 1992, WEINHEIM, BRD Seiten 2795 - 2799 T. DOBNER ET AL. 'Differentiation-specific expression of a novel G protein-coupled receptor from Burkitt's lymphoma.'
- CELLULAR AND MOLECULAR BIOLOGY, Bd.40, Nr.3, Mai 1994, NOISY-LE-GRAND, FRANKREICH Seiten 413 - 419 T. EMRICH ET AL. 'Transmembrane topology of the lymphocyte-specific G-protein-coupled receptor BLR1: Analysis by flow cytometry and immunocytochemistry.'
- BLOOD, Bd.84, Nr.3, 1. August 1994, NEW YORK NY, VSA Seiten 830 - 840 R. FÖRSTER ET AL. 'Expression of the G-protein-coupled receptor BLR1 defines mature, recirculating B cells and a subset of T-helper memory cells.'
- BIOCHEMICAL AND BIOPHYSICAL RESEARCH COMMUNICATIONS, Bd.196, Nr.3, 15. November 1993, DULUTH MN, VSA Seiten 1496 - 1503 R. FÖRSTER ET AL. 'A general method for screening mAbs specific for G-protein coupled receptors as exemplified by using epitope tagged BLR1-transfected 293 cells and solid phase cell ELISA.'

## Beschreibung

Die Erfindung betrifft einen monoklonalen Antikörper hinterlegt unter DSM ACC 2153 gegen Leukozyten-spezifische G-Protein gekoppelte Rezeptoren, Verfahren zu seiner Herstellung und seine Verwendung sowie ihn enthaltende Kits. Ferner betrifft die Erfindung ein Verfahren zur Herstellung eines monoklonalen Antikörpers gegen G-Protein gekoppelte Rezeptoren.

Es ist bekannt, daß bei der Transduktion von Signalen in einem Organismus Rezeptoren eine entscheidende Rolle spielen. Nach Bindung der Liganden an Zellmembran-integrierte Rezeptoren wird das Signal über verschiedene Mechanismen intrazellulär weitergeleitet und verarbeitet. Es gibt verschiedene Familien dieser Rezeptoren, die sich durch bestimmte Strukturmerkmale unterscheiden. Die sogenannten G-Protein-gekoppelten Rezeptoren, international abgekürzt als GCR (G protein-coupled receptors), werden in eine Familie zusammengefaßt. Das G-Protein ist ein heterotrimärer GTP-bindender Proteinkomplex, der aus den drei Untereinheiten G-alpha, G-beta und G-gamma aufgebaut ist. Es reguliert zelluläre Aktivitäten durch Austausch von GDP gegen GTP an seiner alpha-Untereinheit und aktiviert bzw. inaktiviert somit eine Reihe von Effektoren, wie Adenylylcyclasen, Phosphodiesterasen, Phospholipasen und Ionenkanäle. Als Vertreter von G-Protein-gekoppelten Rezeptoren werden Rezeptoren für Hormone und Neurotransmitter angesehen. Zu ihnen gehören adrenerge und muscarinerge Rezeptoren wie auch Rezeptoren für Dopamin, der Substanz P, Thyreotropin, Morphin u. a..

Desweiteren ergeben sich zunehmend Hinweise, daß G-Protein-gekoppelte Rezeptoren auch an der Regulation der Aktivierung, Hemmung, Migration und Zell-Zell-Interaktion immunkompetenter Zellen beteiligt sind. Es ist bekannt, daß die Aktivierung von Leukozyten durch Entzündungsmediatoren, wie formyl-MLP, Anaphylatoxin C5a, Prostaglandine, Interleukin-8, MIP-1α und MIP-1β sowie RANTES, ebenfalls über G-Protein-gekoppelte Rezeptoren erfolgt. Eine Blockierung dieser Rezeptoren würde eine Entzündungshemmung bewirken. Mittel hierfür wurden jedoch bisher noch nicht gefunden.

Der Erfindung liegt somit die Aufgabe zugrunde, Mittel bereitzustellen, mit denen Leukozyten-spezifische G-Protein gekoppelte Rezeptoren blockiert werden können.

Erfindungsgemäß wird dies durch Bereitstellung des monoklonalen Antikörpers gegen Leukozyten-spezifische G-Protein gekoppelte Rezeptoren (L-GCR) DSM ACC 2153 erreicht. Dieser Antikörper ist durch folgende Verfahrensschritte erhältlich:
(a) Einführung einer L-GCR-codierenden DNA in Zellen und Expression von L-GCR,
(b) Immunisierung eines Tieres mit L-GCR-exprimierenden Zellen von (a), und
(c) Fusion von Milzzellen des immunisierten Tieres von (b) mit Myelomzellen und Erhalt von monoklonale L-GCR-Antikörper-produzierenden Hybridomzellen.

Die L-GCR-codierende Nukleinsäure kann eine DNA, insbesondere eine genomische oder eine cDNA sein. Ferner kann sie eine RNA sein. Die Nukleinsäuren können durch übliche aus der Literatur bekannte Verfahren bereitgestellt werden (vgl. z.B. Maniatis et al., Molecular Cloning: A Laboratory Manual, Cold Spring Harbor Laboratory (1982); Lipp et al., Eur. J. Immunol. 22 (1992), 2795-2799).

Erfindungsgemäß können in die L-GCR-codierende Nukleinsäure Modifikationen, wie Additionen, Deletion und/oder Substitution von ein oder mehr Basen eingeführt werden. Additionen umfassen Markersequenzen, die z.B. an das 5'-Ende oder 3'-Ende der L-GCR-codierenden Nukleinsäure fusioniert werden. Solche Markersequenzen sind z.B. Codons, die für ein Protein bzw. Proteinfragment codieren, gegen das ein monoklonaler Antikörper existiert. Mit letzerem kann die Expression des Proteins bzw.

Proteinfragments und damit auch die von L-GCR nachgewiesen werden (vgl. von Zastrow und Kobilka, J. Biol. Chem. 267 (1992), 3530-3538; von Zastrow et al., J. Biol. Chem. 268 (1993), 763-766; T. Emrich, M. Lipp, unveröffentlicht). Markersequenzen können auch in die für L-GCR codierende Nukleinsäure, z.B. in die für die extra-zellulären oder intra-zellulären Domänen codierenden Sequenzen, eingefügt werden. Weitere Additionen sind Sequenzen, die eine Lokalisierung von L-GCR in der Zellmembran von L-GCR-exprimierenden Zellen gewährleisten. Solche Sequenzen können für Signalpeptide, Membranproteine oder Fragmente davon codieren. Üblicherweise werden sie an das 5'-Ende der L-GCR-codierenden Nukleinsäure fusioniert.

Erfindungsgemäß kann die L-GCR-codierende Nukleinsäure in einer Expressionseinheit vorliegen. Diese umfaßt die für die Transkription und Translation der L-GCR-codierenden Nukleinsäure notwendigen Sequenzen, wie Promotor-, Enhancer-, Ribosomenbindungs-, Transkriptionsonsstart- und stop- sowie Translationsstart- und stop-Sequenzen. Die Expressionseinheit kann ferner in einem Vektor vorliegen. Dies kann auch ein Virus sein. Dem Fachmann ist bekannt, welche der Sequenzen der Expressionseinheit in welcher Anordnung notwendig sind, um L-GCR in bestimmten Zellen exprimieren zu können. Desweiteren weiß der Fachmann, welche Vektoren für welche Zellen geeignet sind.

Erfindungsgemäß wird die L-GCR-codierende Nukleinsäure in Zellen eingeführt, um L-GCR zu exprimieren. Hierfür können übliche aus der Literatur bekannte Verfahren und Bedingungen zur Transfektion von Zellen mit Nukleinsäure angewandt werden. Beispiele sind das Calciumphosphat-Präzipitations-, das DEAE-Dextran-, das Elektroporations- und das Lipidvesikel-Verfahren. Günstigerweise wird das Calciumphosphat-Präzipitationsverfahren angewandt, wobei etwa 1-2x10⁶ Zellen mit etwa 15 µg L-GCR-codierender Nukleinsäure transfiziert werden. Als Zellen können eukaryotische und prokaryotische Zellen verwendet werden. Bevorzugt sind eukaryotische Zellen, insbesondere Säugetier-, z.B. CHO-, COS- und 293-Zellen, Hefe- und Insektenzellen. Die Zellen sind dem Fachmann bekannt und allgemein erhältlich. Die Expression von L-GCR kann indirekt nachgewiesen werden, z.B. durch Nachweis der Expression von Markersequenzen, die von der L-GCR-codierenden Nukleinsäure umfaßt werden, oder durch Ligandenbindung. Der Nachweis kann mittels üblicher, aus der Literatur bekannter Verfahren, z.B. der Immunfluoreszenz, der Immunenzymologie, der ELISA-Technik, der Durchflußcytometrie und den Ligandenbindungstests erfolgen.

Erfindungsgemäß wird ein Tier mit L-GCR-exprimierenden Zellen immunisiert. Hierfür können übliche aus der Literatur bekannte Bedingungen gewählt werden. Günstigerweise werden ca. 3x10⁷-Zellen einem Tier zweimal verabreicht, wobei etwa 28 Tage zwischen den Verabreichungen liegen. Als Tiere können übliche Versuchstiere, insbesondere Ratten, Hamster, Kaninchen und Mäuse, verwendet werden. Als besonders günstig sind Ratten zu erwähnen.

Erfindungsgemäß werden dem immunisierten Tier Milzzellen entnommen. Dies kann in üblicher und bekannter Weise erfolgen. Günstigerweise werden Milzzellen ca. 60 Stunden nach der zweiten Verabreichung des Antigens dem Tier entnommen. Sie werden dann mit Myelomzellen mittels PEG fusioniert. Hierfür können übliche aus der Literatur bekannte Bedingungen verwendet werden. Günstigerweise werden als Myelomzellen solche der Mäuse-Myelomzellinie X63-Ag8.653 (Kearney et al., J. Immunol. 123 (1979), 1548-50) verwendet und das Verhältnis der Milzzellen zu den Myelomzellen beträgt etwa 1 : 3. Etwa 1 Woche nach Fusion werden die Überstände der fusionierten Zellen auf Antikörperproduktion getestet. Hierfür können übliche aus der Literatur bekannte Verfahren durchgeführt werden. Günstigerweise wird eine Durchflußcytometrie durchgeführt. In ihr werden Vergleichsmessungen der Hybridomzell-Überstände auf Zellen durchgeführt, die mit L-GCR-codierender Nukleinsäure transfiziert worden bzw. unverändert (nicht transfiziert) sind. Antikörper-produzierende Hybridomzellen werden dann durch übliche aus dem Stand der Technik bekannte Verfahren, z.B. der Limiting Dilution-Technik, kloniert.

Eine solche den Antikörper RF8B2 produzierende Hybridomzelle wurde am 8. Sept. 1993 bei der Deutschen Sammlung von Mikroorganismen und Zellkulturen, Mascherodeweg 1b, 38124 Braunschweig (DSM) unter der Hinterlegungsnummer DSM ACC 2153 hinterlegt.

Der erfindungsgemäße L-GCR-Antikörper eignet sich für diagnostische Maßnahmen, insbesondere für die Bestimmung des Immunstatus eines Patienten. Hierfür wird er z.B. mit Fluorochromen oder Biotin markiert und zusammen mit anderen markierten, allgemein erhältlichen T-Zell- bzw. B-Zell-spezifischen Antikörpern und Körperflüssigkeiten des Patienten inkubiert. Es wird der Anteil an T-Zellen, Gedächtnis-T-Zellen, T-Helfer₁-und T-Helfer₂-Zellen bzw. rezirkulierenden, nicht aktivierten B-Zellen bestimmt. Dies ermöglicht eine Aussage über den Immunstatus des Patienten. Ferner kann der erfindungsgemäße L-GCR-Antikörper zum Nachweis von Erkrankungen, insbesondere von Tumoren, Leukämien, Lymphomen, Immunschwächen und Autoimmunerkrankungen verwendet werden.

Desweiteren eignet sich der erfindungsgemäße L-GCR-Antikörper für therapeutische Maßnahmen. Durch Bindung an den Rezeptor beeinflußt er die Bindung des physiologischen Liganden oder führt durch die Bindung selbst zu einer Aktivierung oder Hemmung des Rezeptors. Der erfindungsgemäße Antikörper eignet sich zur Hemmung von Entzündungen und zur Reduzierung der Bund T-Zellaktivierung. Er kann ferner die Migration und Interaktion von Zellen beeinflussen. Desweiteren eignet sich der erfindungsgemäße Antikörper, allein oder in Kombination mit anderen Antikörpern (z.B. gegen Adhäsionsmoleküle) oder Therapeutika, eine Tumormetastasierung zu unterdrücken. Durch Hemmung der Ligandenbindung an L-GCR wird eine Reduzierung der Aktivierung von Adhäsionsmolekülen bewirkt, was entscheidend die Tumormetastasierung hemmt.

Darüberhinaus kann der die erfindungsgemäße Antikörper mit bekannten Zelltoxinen, z.B. Ricin, oder Therapeutika gekoppelt werden, wodurch entartete Zellen selektiv behandelt werden können. Nach Bindung des Antikörpers kommt es zur Internalisierung des Rezeptor-Komplexes und mithin zur effektiven Aufnahme des gekoppelten Toxins oder der Therapeutika. Der vorstehend genannte Antikörper RF8B2 ist hierfür besonders geeignet, da er der Subklasse IgG_{2b} angehört, die bekannterweise äußerst gering immunogen ist.

Der erfindungsgemäße Antikörper wird für die Verabreichung in therapeutischen Maßnahmen, z.B. als Medikament, in üblicher Weise konfektioniert.

Erfindungsgemäß wird auch ein Kit bereitgestellt, der sich zur Durchführung vorstehend angesprochener diagnostischer Maßnahmen eignet. Ein solcher Kit enthält
- markierten L-GCR-Antikörper, übliche Waschpuffer und ggfs. ein der Markierung entsprechendes Substrat sowie ggfs. einen weiteren markierten Antikörper. oder
- markierten L-GCR-Antikörper und markierten T-Zell-spezifischen Antikörper und/oder B-Zell-spezifischen Antikörper sowie übliche Waschpuffer und ggfs. ein der Markierung entsprechendes Substrat sowie ggfs. einen weiteren markierten Antikörper.

Ergänzend wird ausgeführt, daß in Schritt (a) des vorstehenden Verfahrens zur Herstellung des L-GCR-Antikörperns die L-GCR-codierende Nukleinsäure durch eine Nukleinsäure ersetzt werden kann, die für einen anderen G-Protein-gekoppelten Rezeptor (GCR), z.B. einen Hormon- oder Neurotransmitter-Rezeptor codiert. Dies führt dann zur Expression von GCR in Zellen und weiter nach Immunisierung eines Tieres mit solchen Zellen und Fusion von Milzzellen des immunisierten Tieres mit Myelomzellen zu Hybridomzellen, die monoklonale GCR-Antikörper produzieren. Erfindungsgemäß wird also auch ein Verfahren bereitgestellt, das sich zur Herstellung monoklonaler Antikörper gegen G-Protein-gekoppelte Rezeptoren eignet.

Schließlich wird darauf hingewiesen, daß anstelle der Schritte (a) und (b) des vorstehenden Verfahrens zur Herstellung von L-GCR- bzw. GCR-Antikörpern die Nukleinsäure direkt in ein Tier zur Expression von L-GCR bzw. GCR eingeführt werden kann, wodurch das Tier immunisiert wird. Ferner kann anstelle der Fusion von Schritt (c) ein anderes aus dem Stand der Technik bekanntes Verfahren zur Immortalisierung von Milzzellen verwendet werden.

Die folgenden Beispiele erläutern die Erfindung.

### Beispiel 1

### Konstruktion eines Plasmids zur Expression von L-GCR in eukaryotischen Zellen.

Eine für L-GCR-codierende cDNA (vgl. Lipp et al., Eur. J. Immunol. (1992) 22, 2795-2799) wurde in die Restriktionsschnittstellen EcoRI und Xbal des bekannten und allgemein erhältlichen prokaryotischen Klonierungsvektors pBluescript II KS+ eingefügt, wobei zuvor folgende Modifizierungen vorgenommen wurden. Im 5'-untranslatierten Bereich wurde mittels eines synthetischen Oligonukleotids 10 Basenpaare vor dem Translationsstartcodon eine Schnittstelle für die Restriktionsendonuklease EcoRI eingefügt und der Bereich vor dem Translationsstartcodon derart verändert, daß eine effiziente Initiation der Translation erfolgt (vgl. Kozak M., Cell (1986) 44, 283-292). Das Tranlationsstopcodon wurde mittels Polymerasekettenreaktion entfernt und die L-GCR-codierende DNA mit einem synthetischen Oligonukleotid derart rekombiniert, daß auf Proteinebene an den Carboxy-Terminus des L-GCR-Proteins die Aminosäuresequenz PGGSGPEQKLI SEEDLL fusioniert wurde. Die Sequenz der letzten 11 Aminosäuren stammt aus dem MYC-Protein und wird vom monoklonalen Antikörper 9E10 erkannt (vgl. Bishop, J.M. et al., Mol.Cell.Biol.5 1985),3610-3616; Munro S., Cell 46 (1986), 291-300), während die ersten sechs Aminosäuren als Abstandshalter dienen, um eine ungestörte Faltung des MYC-Epitops zu ermöglichen. Die erhaltene rekombinante DNA wurde in die Restriktionsschnittstellen HindIII und Xbal des bekannten und allgemein erhältlichen eukaryotischen Expressionsvektors Rc/CMV, der die Expression mittels eines heterologen CMV-Promotors ermöglicht, eingefügt. Es wurde das mit pBLR1-MYC_{c} bezeichnete Plasmid erhalten.

### Beispiel 2

### Expression von L-GCR in eukaryotischen Zellen.

Zur Expression von L-GCR in Eukaryoten wurde die humane embryonale Nierentumorzellinie 293 (ATCC CRL 1573) verwendet. Hierzu wurden ca. 1-2 x 10⁶ Zellen mit 15 µg des Plasmids von Beispiel 1 mittels calciumphosphat-Präzipitationstechnik transfiziert. Die Plasmid-DNA wurde zuvor aus den transformierten Bakterien über Ionenaustauschchromatographie isoliert. Zur Etablierung stabil exprimierender Zellinien wurden Antibiotika-resistente Zellen des Transfektionsansatzes durch Zusatz von Neomycin (200 µg/ml) angereichert und in der Expression des L-GCR-MYC-Fusionsproteins wurden positive Zellinien durch Einzelzellverdünnung isoliert. Die Kontrolle der Expression des Fusionsproteins erfolgte sowohl bei transient als auch bei stabil transfizierten Zellen mittels Durchflußcytometrie an permeabilisierten Zellen unter Verwendung des monoklonalen Antikörpers 9E10.

### Beispiel 3

### Herstellung monoklonaler Antikörper

### Immunisierung einer Ratte

Weibliche, acht Wochen alte, Lou/C Ratten wurden mit je 3x10⁷ lebenden L-GCR-transfizierten 293-Zellen immunisiert. Nach 28 Tagen erfolgte der Booster mit der gleichen Menge des Immunogens.

### Zellfusion

60 Stunden nach der letzten Immunogenverabreichung wurde die Fusion in üblicher Weise durchgeführt (vgl. z.B. Köhler G. und C. Milstein, Nature 256 (1975), 495-497). Hierbei wurde der immunisierten Ratte unter sterilen Bedingungen die Milz entnommen, durch ein Sieb zerrieben und die gewonnenen Zellen wurden im Verhältnis 1:3 mit Zellen der Mäuse-Myelomzellinie X63-Ag8.653 (Kearney et al., J. Immunol. 123 (1979) 1548-1550) mit Hilfe von PEG fusioniert. Eine Woche nach der Aussaat in Flachbodenplatten in HAT-Selektivmedium wurde das Koloniewachstum protokolliert und die Zellkulturüberstände koloniehaltiger Vertiefungen wurden im Durchflußcytometer (vgl. nachstehend) vergleichend auf stabilen L-GCR-exprimierenden 293 Zellen und normalen 293 Zellen auf das Vorhandensein L-GCRspezifischer Antikörper getestet. Nur solche Antikörper, die an transfizierten Zellen gebunden haben, nicht jedoch an normalen 293 Zellen, wurden nach der Limiting dilution-Technik kloniert, weitervermehrt, auf transient transfizierten 293 Zellen und schließlich auf peripheren Leukozyten getestet.

### Durchflußcytometrie

Das Vorhandensein von L-GCR-Antikörpern wurde mittels Durchflußcytometrie getestet. Als Testsystem wurden vergleichende Messungen an L-GCR-transfizierten und unveränderten 293 Zellen durchgeführt. Je 25 µl Hybridomzellen-Überstand wurden mit 25 µl der jeweiligen Zellsuspension (1x10⁷/ml) für 25 min bei RT inkubiert. Nach Waschen in PBS (4% FKS, 5 mM EDTA) wurden die Zellen mit einer 1:160 Verdünnung eines polyklonalen Ziegeanti-Ratte-FITC Konjugates versetzt. Nach 25-minütiger Inkubation und anschließendem Waschen erfolgte der Nachweis einer spezifischen Bindung mittels eines FACScan Durchflußcytometers. Je Probe wurden 5000 Zellen gemessen und die Fluoreszenzintensität der Zellpopulation wurde analysiert. Durch den Vergleich der Fluoreszenzintensität von transfizierten und unveränderten 293 Zellen konnten solche Antikörper identifiziert werden, die spezifisch an L-GCR binden.

### Bindungsstudien des erfindungsgemäßen Antikörpers RF8B2

Vorstehend angegebene 293 Zellen wurden mit L-GCR codierender DNA in üblicher Weise transfiziert bzw. nicht transfiziert. Letztere Zellen wurden als mock-transfiziert bezeichnet. Der erfindungsgemäße Antikörper RF8B2 wurde in üblicher Weise mit Biotin markiert und den Zellen zugegeben. Es zeigte sich, daß RF8B2 an transfizierte 293 Zellen bindet, nicht jedoch an mock-transfizierte (vgl. Fig., A).

Desweiteren wurden periphere Leukozyten des Blutes über Ficoll gereinigt und mit dem T-Zell-spezifischen Antikörper CD4-FITC (B) oder CD8-FITC (C) bzw. mit dem B-Zell-spezifischen Antikörper CD19-FITC (D) angefärbt. Anschließend wurde der Biotinmarkierte Antikörper RF8B2 zugegeben. Es zeigte sich, daß 100% der B-Zellen (CD19-positiv) (D) diesen Antikörper binden. Hingegen wird RF8B2 nur von ca. 14% der T-Helfer-Zellen (CD4-positiv) (B), und ca. 2% der zytotoxischen T-Zellen (CD8-positiv) (C) gebunden (vgl. Fig., B, C und D). Dies zeigt die Verwendbarkeit von RF8B2, eine unterschiedlich starke Expression von L-GCR aufzuzeigen.

### Produktion und Reinigung von Antikörpern

| | | |
|---|---|---|
| Probenpuffer | pH 7,4 | PBS |
| Elutionspuffer | pH 2,5 | 0,05 M Glycin |
| | | 0,05 M NaCl |
| 10fach Tris-HCl-Puffer | pH 8,6 | 0,5M Tris |
| | | 1,5M NaCl |

Die Hybridomzellen wurden im RPMl mit 10% IgG-freiem FKS vermehrt, alle 2-3 Tage 1:3 geteilt und der Zellkulturüberstand wurde gesammelt. Zur Abtrennung der monoklonalen Antikörper aus dem Zellkulturüberstand wurde die Protein G-Affinitätschromatographie verwendet (vgl. Björck und Kronvall, J. Immunol. 132 (1984) 969-974). Hierzu wurde 1 g Protein G Sepharose 4 Fast Flow nach Quellung in eine Chromatographiesäule gepackt und mit PBS äquilibriert. 200 ml des zu reinigenden Überstandes wurden nach Mikrofiltration auf die Säule aufgebracht. Ein Wechsel von PBS auf Elutionspuffer löste die an die Sepharose gebundenen Antikörper. Die dem Extinktions-Peak entsprechenden Eluatfraktionen wurden gegen PBS-dialysiert.

### Markierung von Antikörpern

| Biotin-Markierung: | | |
|---|---|---|
| Kupplungspuffer | pH 7,4 | PBS |
| Biotinpräparat | NHS-LC-Biotin | |

Hierbei wurde die Kupplung mit Abwandlungen wie beschrieben, ausgeführt (vgl. z.B. Peters et al., Monoklonale Antikörper, Herstellung und Charakterisierung, Springer Verlag, Berlin (1985)). Protein G gereinigte Eluate wurden auf 2 mg/ml eingestellt und gegen Kupplungspuffer 24 Stunden dialysiert. Zur Reaktion wurden 50 µl Biotiniösung (8 mg Biotin in 1 ml DMF) zu 1,0 ml MAk-Lösung gegeben und 90 min bei Zimmertemperatur inkubiert. Nach Dialyse gegen PBS- und Sterilzentrifugation wurden die Konjuagte bei -20°C gelagert.

| FITC-Konjugate: | | |
|---|---|---|
| Kupplungspuffer | pH 9,5 | 0.1M NaHCO₃ |

FITCpräparat Fluoresceinisothiocyanat,
Protein G gereingte Eluate wurden auf 2 mg/ml eingestellt und mit 1/10 Vol. 10fach Kupplungspuffer versetzt. Zur Reaktion wurden 60 µl FITClösung (1mg FITC in 1 ml Kupplungspuffer) zu 1,0 ml mAk-Lösung gegeben und über Nacht bei 4°C inkubiert.

Nach Dialyse gegen PBS- und Sterilzentrifugation wurden die Konjugate bei -20°C gelagert.

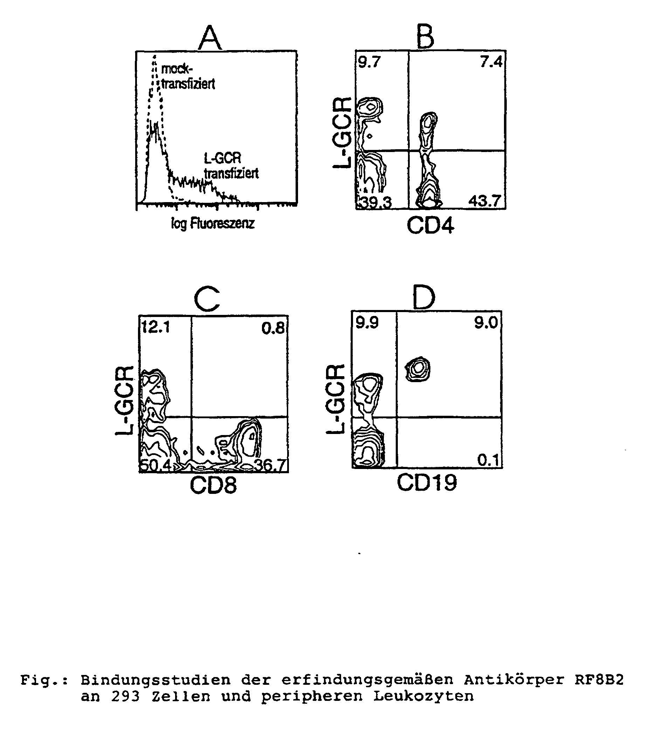

## Patentansprüche

1. Monoklonaler Antikörper gegen Leukozyten-spezifische G-Protein gekoppelten Rezeptor (L-GCR) DSM ACC 2153, erhalten durch folgende Verfahrensschritte:
a) Einführung einer L-GCR-codierenden Nukleinsäure in Zellen und Expression von L-GCR
b) Immunisierung eines Tieres mit L-GCR-exprimierenden Zellen von a) und
c) Fusion von Milzzellen des immunisierten Tieres von b) mit Myelomzellen.

2. Verwendung des Antikörpers nach Anspruch 1 zur Herstellung von Mitteln für diagnostische und therapeutische Zwecke.

3. Verwendung nach Anspruch 2, **dadurch gekennzeichnet, daß** die Mittel für diagnostische Maßnahmen zur Bestimmung des Immunstatus eines Patienten sowie zum Nachweis von Tumoren, Leukämien, Lymphomen, Immunschwächen und Autoimmunerkrankungen verwendet werden.

4. Verwendung nach Anspruch 2, **dadurch gekennzeichnet, daß** die Mittel für therapeutische Maßnahmen zur Beeinflussung der Funktion von Blutzellen, z.B. zur Hemmung von Entzündungen und zur Unterdrückung einer Tumormetastasierung verwendet werden.

5. Kit, enthaltend
- markierten Antikörper nach Anspruch 1, übliche Waschpuffer und ggf. ein der Markierung entsprechendes Substrat sowie ggf. einen weiteren markierten Antikörper oder
- markierten Antikörper nach Anspruch 1 und T-Zell-spezifischen Antikörper und/oder B-Zell-spezifischen Antikörper sowie übliche Waschpuffer und ggf. ein der Markierung entsprechendes Substrat sowie ggf. einen weiteren markierten Antikörper.

6. Verfahren zur Herstellung des monoklonalen Antikörpers gegen Leukozyten-spezifische G-Protein gekoppelten Rezeptor (L-GCR) DSM ACC 2153, **gekennzeichnet durch** folgende Verfahrensschritte:
a) Einführung einer L-GCR-codierenden Nukleinsäure in Zellen und Expression von L-GCR
b) Immunisierung eines Tieres mit L-GCR-exprimierenden Zellen von a) und
c) Fusion von Milzzellen des immunisierten Tieres von b) mit Myelomzellen und Erhalt von monoklonale L-GRC-Antikörper produzierenden Hybridomzellen.

## Claims

1. Monoclonal antibodies against leukocyte-specific G-protein coupled receptor (L-GCR) DSM ACC 2153, obtained by the following procedural steps:
a) introduction of an L-GCR coding nucleic acid into cells and expression of L-GCR
b) immunisation of an animal with L-GCR expressing cells of a) and
c) fusion of spleen cells of the immunised animal of b) with myeloma cells.

2. Use of the antibody according to Claim 1 for the production of agents for diagnostic and therapeutic purposes.

3. Use according to Claim 2, wherein the agents for diagnostic measures are used for the determination of the immune status of a patient as well as for detection of tumours, leukaemias, lymphomas, immune weaknesses and auto-immune diseases.

4. Use according to Claim 2, wherein the agents for therapeutic measures are used to influence the function of blood cells, e.g. to inhibit inflammations and to suppress tumour metastasisation.

5. Kit, containing
marked antibody according to Claim 1, customary washing buffers and, if need be, a substrate corresponding to the marking and also, if need be, a further marked antibody or
marked antibody according to Claim 1 and T-cell-specific antibody and/or B-cell-specific antibody as well as customary washing buffers and, if need be, a substrate corresponding to the marking and also, if need be, a further marked antibody.

6. Method for the production of a monoclonal antibody against leukocyte-specific G-protein coupled receptor (L-GCR) DSM ACC 2153, wherein there exist the following procedural steps:
a) introduction of an L-GCR coding nucleic acid into cells and expression of L-GCR
b) immunisation of an animal with L-GCR expressing cells of a) and
c) fusion of spleen cells of the immunised animal of b) with myeloma cells and receipt of monoclonal hybridoma cells producing L-GRC antibodies.

## Revendications

1. Anticorps monoclonaux contre le récepteur (L-GCR) DSM ACC 2153 lié à la protéine G spécifique aux leucocytes, produits par les étapes de processus suivantes:
a) Introduction d'un acide nucléique codé L-GCR dans des cellules et expression de L-GCR.
b) Immunisation d'un animal avec des cellules exprimant le L-GCR de a) et
c) Fusion de cellules de la rate de l'animal immunisé de b) avec des cellules de myélome.

2. Emploi de l'anticorps selon la revendication 1 pour la fabrication de produits destinés à réaliser un diagnostic et un traitement.

3. Emploi selon la revendication 2 qui se **caractérise par le fait que** les produits seront utilisés à des fins diagnostiques pour déterminer l'état d'immunité d'un patient ainsi que la présence de tumeurs, de leucémies, de lymphomes, de déficiences immunitaires et de maladies auto-immunes.

4. Emploi selon la revendication 2 qui se **caractérise par le fait que** les produits seront utilisés à des fins thérapeutiques pour influencer les fonctions des cellules sanguines, par ex. pour inhiber des inflammations et pour réprimer des métastases de tumeurs.

5. Kit, composé d'
- anticorps marqués selon la revendication 1, du tampon de lavage usuel et le cas échéant d'un substrat correspondant au marquage ainsi que le cas échéant d'un autre anticorps marqué ou
- anticorps marqués selon la revendication 1 et d'anticorps spécifiques aux lymphocytes T et/ou d'anticorps spécifiques aux lymphocytes B ainsi que du tampon de lavage usuel et le cas échéant d'un substrat correspondant au marquage ainsi que le cas échéant d'un autre anticorps marqué.

6. Procédé de fabrication de l'anticorps monoclonal contre le récepteur (L-GCR) DSM ACC 2153 lié à la protéine G spécifique aux leucocytes, se caractérisant par les étapes suivantes:
a) Introduction d'un acide nucléique codé L-GCR dans des cellules et expression de L-GCR.
b) Immunisation d'un animal avec des cellules exprimant le L-GCR de a) et
c) Fusion de cellules de la rate de l'animal immunisé de b) avec des cellules de myélome et obtention d'hybridomes produisant des anticorps monoclonaux L-GRC.
